# EUROPEAN PATENT SPECIFICATION

(11) **EP 2 459 327 B1**
(45) Date of publication and mention of the grant of the patent: **10.09.2014**
(21) Application number: 10740370.1
(22) Date of filing: 14.07.2010
(51) Int. Cl.: B08B 9/28, A61L 2/22

(54) **WASHING AND STERILIZING UNIT**
Wasch- und Sterilisationseinheit
Unité de lavage et de stérilisation

(30) Priority: 28.07.2009 EP 09166536
(43) Date of publication of application: 06.06.2012
(73) Proprietor: Koninklijke Philips N.V., 5656 AE Eindhoven (NL)
(72) Inventor: SLOOT, Eric, Mark, NL-5656 AE Eindhoven (NL); KOOIJKER, Klaas, NL-5656 AE Eindhoven (NL); WIJMA, Willem, Sjouke, NL-5656 AE Eindhoven (NL)
(74) Representative: Coops, Peter
(86) International application number: PCT/IB2010/053214
(87) International publication number: WO 2011/013022

(56) References cited:
- WO-A-94/18874
- WO-A-03/071910
- WO-A-2004/023967
- US-A- 4 368 747

## Description

### FILED OF THE INVENTION

The present invention relates to a unit for washing and/or sterilizing baby care components such as a baby bottle.

Babies are particularly susceptible to infections and diseases due to their underdeveloped immune system. Typically, antibodies may be transferred from the mother to the baby via breast feeding thereby enhancing their resistance; however this is generally not the case for formula fed babies. Therefore, it is particularly important to maintain baby care products, such as baby bottles and dummies, clean and sterile during the early months of a baby's development.

### BACKGROUND OF THE INVENTION

Various units for washing and sterilizing baby care components are known, however the configurations of conventional units are inconvenient in that they require a generous amount of space around the unit in order to open the units to access a baby care component receiving space in which the components are washed and sterilized. For example, it is known from WO 2007 063 540 A to provide a washing and sterilizing unit wherein baby bottles are cleaned and sterilized within a housing having a lid hingedly connected to a back wall of a base and which is movable about an axis parallel to said base. However, a disadvantage of this configuration is that the lid must be opened in an upward direction by rotating it about its hinge, and so plenty of space is required above the unit in order to access the baby care component receiving space. Furthermore, such a configuration also requires the unit to have a stiff construction and so the manufacturing costs are relatively expensive

Furthermore, it is also desirable for a washing and sterilizing unit to provide easy access to the baby care components space and to allow for the baby care components to be spaced from one another so as to decrease the risk of cross-contamination, see e.g. nearest prior art document WO-A-2004/23967.

### SUMMARY OF THE INVENTION

The present invention seeks to overcome or substantially alleviate the aforementioned problems mentioned above.

Accordingly, the present invention provides a washing unit for washing and/or sterilizing a baby care component comprising a base and a lid, the base having a bottom wall a side wall extending upwardly from the periphery of the bottom wall, wherein a line of closure between the base and the lid at least partially extends in a direction away from the bottom wall, and a rim, the rim comprising a lower portion, wherein the lower portion is positioned so that a baby care component is movable into and out of the unit in a direction substantially parallel to the bottom wall when the lid is removed from the base.

An advantage of the above arrangement is that it enables the lid to be removed from the base substantially in a direction parallel to the bottom wall of the base without the lid contacting baby care components disposed in the unit. This enables the lid to be opened and closed without the need to lift the lid completely up and over interior parts of the unit and baby care components disposed therein. Another advantage is that the manufacturing costs of the unit may be reduced compared to conventional units.

Preferably, the line of closure is formed along a plane which extends at an oblique angle to the bottom wall of the base.

In one embodiment, the unit further comprises engagement means extending along the line of closure such that the lid is engagably mountable to the base, the lid being configured to be removed from the base in a direction parallel to the base once the lid is disengaged from the base.

Conveniently, the side wall of the base has a rim extending there around and the lid comprises a top wall and a lid side wall depending from the top wall, the lid side wall having a brim which is configured to locate against the rim of the base when the lid is engagably mounted to the base.

In one embodiment, the lid side wall extends circumferentially around the top wall and the brim is spaced from said top wall.

In an alternative embodiment, the base further comprises an upper wall extending from an upper end of the base side wall and spaced from the bottom wall, and an edge of the upper wall defines part of the line of closure.

An edge of the top wall of the lid may locate against the edge of the upper wall of the body when the lid is mounted on the body.

Preferably, the engagement means is a labyrinth seal.

Conveniently, the line of closure is a primary line of closure, and the side wall of the base further comprises an upper section and a lower section, wherein a secondary line of closure between the upper section and the lower section extends around the side wall, such that the upper section is removable from the lower section.

In a preferred embodiment, the side wall of the base is removably attachable to the bottom wall of the base.

The base and the lid may together define a baby care component receiving space, and the unit may further comprise a rack disposed in the baby care component receiving space which is rotatable in the space about a vertical axis extending perpendicular to the base.

The unit as described hereinbefore may be cylindrical.

According to another aspect of the invention, there is provided a unit for washing and/or sterilizing a baby care component comprising a base and a lid which together define a baby care component receiving space, the base having a bottom wall and a sidewall extending upwardly from the periphery of the bottom wall, the unit further comprising a rack disposed in the baby care component receiving space which is rotatable in the baby care component receiving space about a vertical axis extending perpendicular to the base.

In an alternative embodiment, a spindle is mounted in the baby care component receiving space and extends along said vertical axis, the rack being mounted to the spindle.

Preferably, the spindle is rotatably mounted in the baby care component space and the rack is fixedly mounted to the spindle such that the rack and the spindle are configured to simultaneously rotate about said vertical axis.

The body may comprise a base and a side wall upstanding from and extending around the base wherein a flange is formed on the side wall extending circumferentially around the baby care component receiving space and a peripheral edge of the rack is disposed to lie on said flange such that it can be rotated about said vertical axis.

Preferred embodiments will now be described, by way of example only, with reference to the accompanying drawings, in which:

### BRIEF DESCRIPTION OF THE DRAWINGS

Figure 1 shows a front view of a unit for washing and/or sterilizing baby bottles;
Figure 2 shows a cross-sectional view of a labyrinth seal of a lid and a body of the unit of Figure 1;
Figure 3 shows a cross-sectional view of the unit of Figures 1 and 2;
Figure 4 shows a cross-sectional view of an alternative embodiment of a unit for washing and/or sterilizing baby bottles;
Figure 5 shows a front view of the unit having a hingedly connected lid;
Figure 6 shows a side view of the unit in Figure 5;
Figure 7 shows a side view of the unit having an irregular line of closure;
Figure 8 shows a front view of the unit having an alternatively configured lid;
Figure 9 shows a front view of a rack, a spindle and a spray arm disposed in the unit of Figure 1;
Figure 10 shows an alternative embodiment of a rack and spindle disposed in the unit of Figure 1;
Figure 11 shows a front view of a support ring disposed in the unit for supporting baby bottles to be positioned in an upright tilting position; and
Figure 12 shows a top view of the support ring of Figure 8 supporting six baby bottles.

### DETAILED DESCRIPTION OF THE EMBODIMENTS

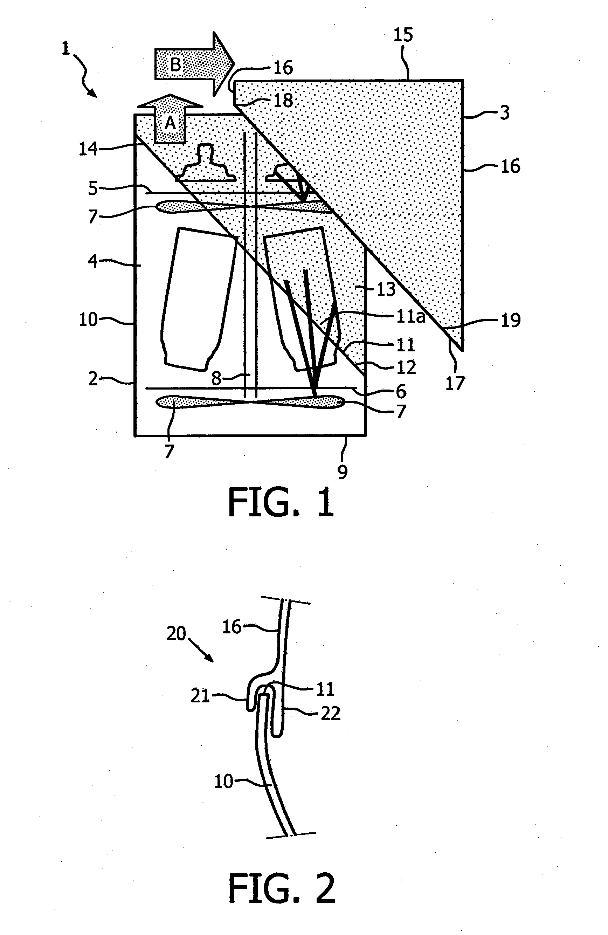

Referring now to the drawings, Figure 1 illustrates a unit 1 for washing and/or sterilizing baby care components. Baby care components include, but are not limited to, baby bottles, baby bottle teats and breast pump accessories. The unit 1 is cylindrical in shape and comprises a base 2 and a lid 3 which together define a baby care components receiving space 4 in which the components are washed, sterilized and/or dried.

Positioned within the unit 1 are upper and lower racks 5, 6 onto which the components such as baby bottles and teats are disposed. Beneath each rack 5, 6 is a pair of spray arms 7 rotatably attached by a low friction mounting to a central, upstanding spindle 8 extending along a vertical longitudinal axis through the unit 1. The spray arms 7 are connected to a water supply and during washing of the enclosed components the spray arms 7 may rotate about the vertical axis of the spindle 8 whilst discharging high pressured water which is injected into the bottles and teats to clean the inside and to also flush the outsides thereof. The hydraulic forces combined with heat rinse the components and remove debris. After washing, hot water is used to sterilize the components and the inside of the unit 1. The water used for sterilizing is generally heated to a temperature above 70°C. It is also envisaged that the present invention can be configured to dry the components after washing and/or sterilization.

The base 2 of the unit 1 comprises a bottom wall 9 and a side wall 10 upstanding from and extending around the periphery of the bottom wall 9. The side wall 10 having a rim 11 to which the lid 3 is removably mountable. The rim 11 is configured to extend in a direction away from the bottom wall 9 such that it is inclined at a non-parallel angle relative to the bottom wall 9 and forms an elliptical shape that has a lower portion 12 and an upper portion 14. The rim 11 defines an opening 11a to the base 2 of the unit 1.

The lid 3 includes a top wall 15 having a peripheral side wall 16 downwardly depending there from, formed with a brim 17 inclined at an angle relative to the top wall 15 such that the brim 17 of the lid 3 has an upper portion 18 and a lower portion 19. In this exemplary embodiment, the lid top wall 15 extends parallel to the bottom wall 9 of the base 2. The brim 17 of the lid 3 is inclined at an angle of similar degree as the rim 11 of the side wall 10 of the base 2, and as the lid 3 is mounted on the base 2, the brim 17 and the rim 11 form a line of closure which extends in a direction away from the bottom wall. Therefore, the brim upper and lower portions 18, 19 engage with respective rim upper and lower portions 14, 12 such that the side wall 16 of the lid 3 and the side wall 10 of base 2 form a continuous side wall of the unit 1. The side wall 16 and top wall 15 of the lid 3 therefore define a section 13 of the baby care component receiving space 4 into which components stored in the baby care component receiving space 4 extend, and so components stored in said space 4 extend above the lower portion 12 of the rim 11 into said section 13 of the baby care component receiving space 4 and the upper portion 14 of the rim extends above components disposed in the baby care component receiving space 4.

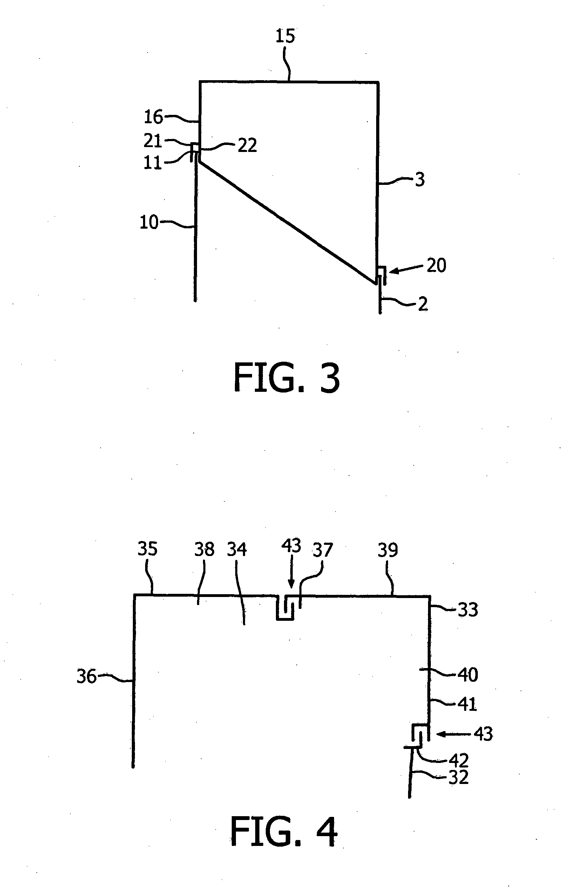

The rim 11 of the base 2 and the brim 17 of the lid 3 engage via a labyrinth seal 20 as can be appreciated from Figures 2 and 3, wherein the brim 17 of the lid 3 is formed with circumferentially extending outer and inner flaps 21, 22 which enclose the rim 11 of the base 2. This configuration prevents water leakage during operation of the unit 1 and enables the lid 3 to be stably mounted onto the base 2.

Removing the lid 3 from the base 2 involves lifting the lid 3 slightly upwards in a vertical direction, as indicated by 'A' in Figure 1, so as to disengage the brim flaps 21, 22 from the rim 11 of the base 2, and then moving the lid 3 in a horizontal direction as indicated by 'B' relative to the bottom wall 9 of the base 2. This reduces the operating space needed around the unit compared to conventional washing and sterilizing units wherein a baby care component receiving space is accessed via a hingedly connected lid rotating about a horizontal or vertical axis or wherein the lid comprises a circumferentially extending sidewall which defines the baby care component receiving space in a 'bucket shaped' arrangement. Therefore, the unit 1 according to the present invention can be stored in a vertically confined space, for example on kitchen work tops having restricted space above the unit due to the presence of cupboards or shelves. Furthermore, as components disposed in the baby care component receiving space 4 do not extend above the upper portion 14 of the rim 11 of the base 2, the lid 3 may be removed from the base 2 in a horizontal direction as described above without contacting and so infecting the sterile components disposed therein. Moreover, when the lid 3 has been removed, the lower portion 12 of the rim 11 of the base 2 provides easy access for a user to retrieve a component from the lower rack 6 without cross-contaminating the remaining washed and sterilized components held within the baby care component receiving space 4.

An alternative embodiment is illustrated in Figure 4. The unit according to this exemplary embodiment is generally the same as the unit described above, and so a detailed description will be omitted. However, in this alternative embodiment, the section of the baby care component receiving space 40 defined by the lid 33 is smaller than in the previously described embodiment. It can be appreciated from Figure 4 that the base 32 comprises an upper wall 35 extending from an upper end of the side wall 36 across a section 38 of the baby care component receiving space 34, and the top wall 39 of the lid 33 extends across the remaining section 40 of the baby care component receiving space 34. The top wall 39 of the lid 33 is formed with a downwardly depending side wall 41 which extends partly around its periphery. The lid 33 has a brim 37 corresponding to and arranged to mount to a rim 42 of the base 32 so that, as the lid 33 is mounted on the base 32, the baby care component receiving space 34 is closed. The brim 37 of the lid 33 and the rim 42 of the base 32 form a line of closure which is inclined at a non parallel angle to the top wall 39 of the lid 33 and a bottom wall (not shown) of the base 32, respectively. The brim 37 of the lid 33 and the rim 42 of the base 32 are provided with a labyrinth seal 43 so as to ensure minimum water leakage and a sterile environment. The configuration of the lid 33 and the base 32 in this embodiment enables the lid 33 to be removed in a similar manner as the embodiment described in reference to Figures 1 and 3. Therefore, the advantages of the previously described embodiment are also realized in this embodiment.

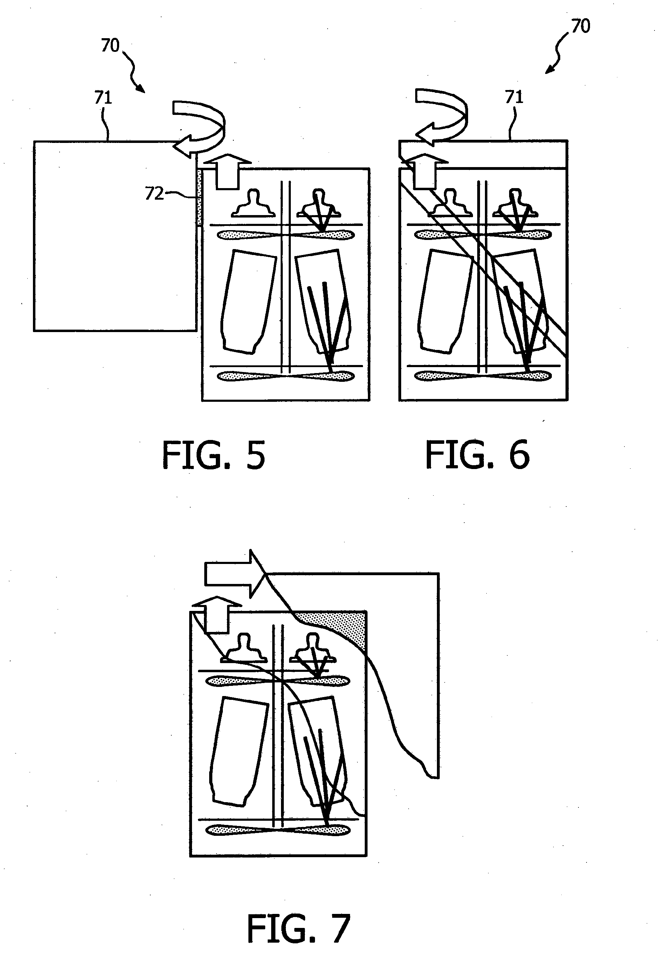

Although, the lid 3, 33 has been described above to be removed by initially moving it in a vertical direction 'A' followed by a horizontal direction 'B', it should be realized that the lid 3, 33 can be removed in a continuous vertical direction or in a direction perpendicular to the inclined line of closure. Furthermore, it will be appreciated that in another embodiment the lid 3, 33 may also be hingedly connected to the base 2, 32. An exemplary configuration of such an embodiment is shown in Figures 5 and 6, wherein the unit 70 comprises a hingedly connected lid 71 is opened by initially moving the lid 71 in a vertical upward direction followed by rotating the lid 71 about a vertical axis of a hinge 72.

Moreover, although in the above embodiments, the rim of the base and brim of the lid are mounted and sealed to each other by means of a labyrinth seal, it will be understood that the arrangement is not limited thereto, and any suitable mounting and sealing arrangement may be used. It is also envisaged that with an alternative sealing arrangement, the lid is removable in a horizontal direction, parallel to the bottom wall 9, to obtain access to the baby care component receiving space without initially moving the lid in a vertical direction to disengage the lid from the base.

It will also be appreciated that although in the above embodiments the line of closure is shown and described extending on a flat plane away from the bottom wall, the line of closure is not limited thereto and that it may form other configurations, such as extending on a curved or irregular plane, as seen in Figure 7.

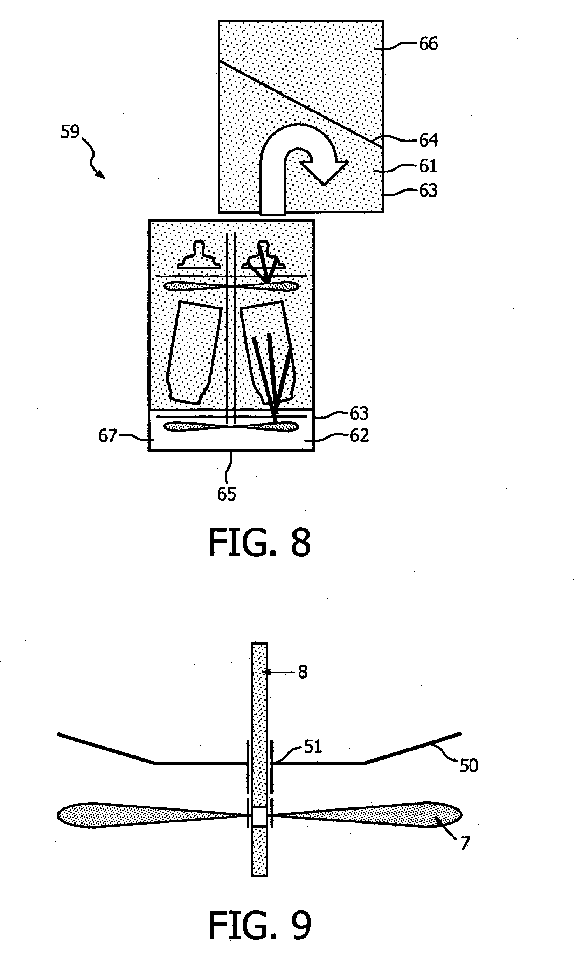

An alternative embodiment of the unit according to the present invention is shown in Figure 8. The unit 59 according to this exemplary embodiment is generally the same as the unit 1 described above, and so a detailed description will be omitted. However, in this alternative embodiment the side wall 63 of the base 67 has upper and lower sections 61, 62 which engage with each other along a plane parallel to the bottom wall 65 of the base 67; the upper section 61 of the side wall 63 extending from the rim 64 towards the lower section 62, the lower section 62 extending upwards from the bottom wall 65. A line of closure is formed between the upper and lower sections 61, 62 of the side wall 63.

The lid 66 and the upper section 61 engage via a labyrinth seal such that the lid 66 is stably mounted on the upper section 61 preventing water from leaking during operation of the unit 59. The lid 66 is removed from the upper section 61 in a similar manner to the lid being removed from the base according to the description of the aforementioned embodiments, such that the lid 66 shown in Figure 8 is removed by initially lifting it from the upper section 61 in a vertical direction followed by a horizontal movement. However, it should be understood that the lid 66 can be removed from the upper section 61 by lifting the lid 66 in a continuous vertical direction.

Although the lid 66 illustrated in Figure 8 is generally the same as the lid 3 in the embodiment described with reference to Figure 1 and 3, it will be appreciated that the lid 66 may be of similar configuration to the lids described with reference to Figures 4 to 7.

The upper section 61 of the side wall 63 of the base is removably attachable to the lower section 62 such that it is attached and removed in a continuous vertical direction over unit interior parts, such as a spindle and racks for holding baby care components which extend in the unit 59. The seal between the upper and lower sections 61, 62 of the side wall 63 of the base 67 may be of a labyrinth seal or any other waterproof seal which also allows for the upper section 61 to be engaged to and disengaged from the lower section 62. The two-part configuration of the side wall 63 provides the user with a choice when accessing the baby care component space by either removing the lid 66 from the upper section 61 of the side wall of the base 67, or by removing the lid 66 and the upper section 61 as one component from the lower section 62 of the side wall 63 of the base 67. Alternatively, the user can first remove the lid and then the upper section 61 of the side wall 63 of the base 67. The different options of removing, as well as attaching, the lid and the upper section make the unit suitable for confined spaces of various spatial restrictions. For example, the unit 59 can easily be operated in vertically restricted spaces by removing the lid 66 in a general horizontal direction. The unit 59 is equally suitable for being placed in horizontally restricted spaces, as the user can then remove the lid 66 and the upper section 61, as one component, from the lower section 62 of the side wall of the base 67 in a continuous vertical direction. Another advantage of the above configuration is that, by dissembling the unit, its inner surfaces and components can easily be accessed and cleaned.

Although in the above embodiment the side wall comprises upper and lower sections, in an alternative embodiment a line of closure is formed between the bottom wall and side wall of the base, such that the side wall is removable from the bottom wall.

It is envisaged that the unit of the above exemplary embodiments is formed from a low cost plastic to provide a cost effective, compact washing and sterilizing unit, however the unit may be formed from other materials such as steel.

Although the aforementioned embodiments have been described as a cylindrical unit it should also be understood that the unit may be of any shape, for example cubic or oval, as well as of any irregular shape, which allows for a lid to be removed in a substantially horizontal and/or vertical direction without the lid contacting components held within the unit and which provides easy access to said components so as to enable a user to retrieve a component without contacting directly or indirectly the remaining components. It will also be understood that the line of closure between the two sections of the side wall or the side wall and the base, as seen from a side view may be straight, curved or irregular.

According to another aspect of the invention, the unit for washing and/or sterilizing baby care components is provided with a rotatable rack. The unit is generally the same as described in the embodiments illustrated in Figures 1 to 3 and so a detailed description will be omitted herein, and components will retain the same reference numerals. The rotatable rack enables baby care components, such as baby bottles, positioned on a lower rack away from the opening 11a of the base 2 to be easily accessed by rotating the rack by hand so that the bottles can be retrieved via the lower portion 12 of the base 2 of the baby care component receiving space, proximate, to the rim 11 of the base 2.

Figure 9 illustrates one embodiment of the rotatable rack 50 wherein the spindle 8 is rotatably mounted to the bottom wall 9 and the rack 50 is formed with a central bore 51 through which the spindle 8 extends, the diameter of the central bore 51 equaling the diameter of the spindle 8 such that the rack 51 is press fitted onto the spindle 8. By pulling or pushing the rack 51, the rack 51 and the spindle 8 rotate simultaneously about the vertical axis defined by the spindle 8. It is also envisaged that the spray arms may be fixedly mounted to the unit such that during operation of the unit, the hydraulic forces of the ejected water from the spray arms cause the rack to rotate to optimize the cleaning and/or sterilization of the baby care components.

Alternatively, the central bore 51 of the rack is configured to be of a diameter which provides high friction between the rack and the spindle so as to maintain the rack 50 in vertical position but allows a user to rotate the rack by hand in a horizontal plane about a fixedly mounted spindle

In another un-illustrated alternative embodiment, the bore has a diameter greater than the diameter of the spindle such that the rack is slidably rotatable about a fixedly mounted spindle and is supported on a collar extending circumferentially around the spindle (not shown). In this embodiment, the spray arms may be rotatable or they may be fixed such that the hydraulic forces exerted by the spray arms cause the rack to rotate. In this case, a second collar is provided on the spindle above the rack, to maintain the rack in its vertical position. Means may also be provided on the spindle guiding the rotatable rack into predetermined positions. For example, the spindle may be provided with "clicks" to locate the rack in six predetermined positions.

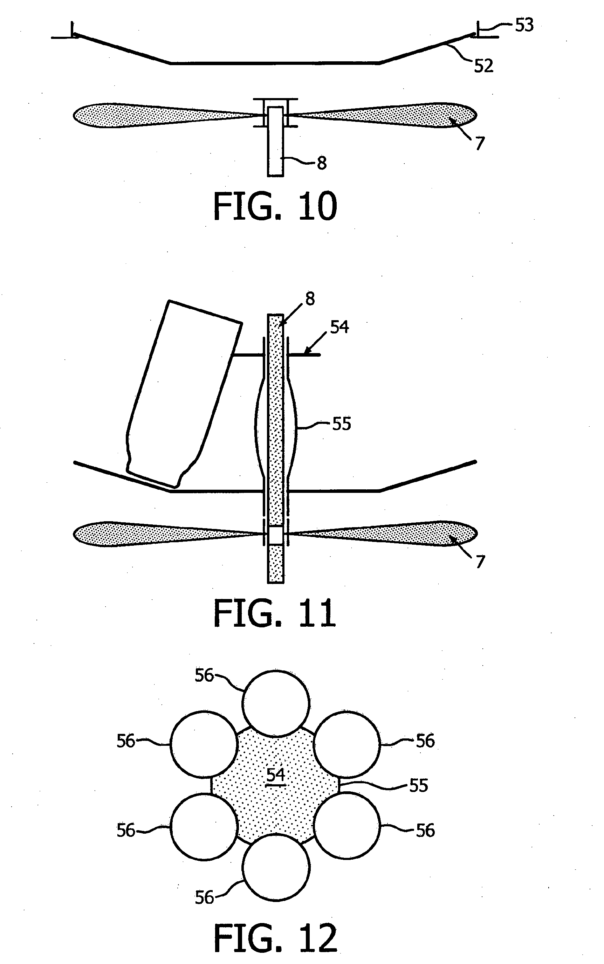

Furthermore, in yet another alternative embodiment, the rack 52 is configured to rest on a cylindrical flange 53 extending circumferentially on the inside of the side wall 10 of the base 2 as illustrated in Figure 10. This allows the user to pull or push the rack 52 so that it rotates about a central vertical axis perpendicular to the bottom wall 9 of the base 2. As the rack 52 does not rely on the spindle 8 for support it is not necessary for the spindle 8 to extend upwards through the unit 1. It should also be understood that the spray arms in this embodiment can either be rotatably or fixedly mounted to the unit. In the case of the spray arms being fixedly mounted, a second cylindrical flange is provided on the inside of the side wall of the base so as to restrict the vertical movement of the rack. However, it is envisaged that the hydraulic forces of the water from the spray arms force the rack to rotate during operation.

In the above embodiments of the unit described as having a spindle, it will be appreciated that the spindle may alternatively comprise a horizontal portion extending from the side wall to the centre of the baby care component receiving space and an upstanding portion which extends from the horizontal portion along a vertical axis of the unit. In this alternative embodiment, it is envisaged that a plurality of spindles may be mounted in the unit, each spindle being provided with spray arms and/or a rack, which engage with the spindle according to the description of any of the aforementioned embodiments.

In order to keep the bottles in an upright tilted position during washing and/or sterilization so as to improve cleaning and drying, a support ring 54 may be provided on the spindle 8 spaced from the rack 5, 50 on which the bottle stands, as shown in Figure 11. In this embodiment, the support ring 54 is connected to the rack 5, 50 via a connector 55 extending between the support ring 54 and the rack 5, 50 so that the support ring 54 rotates simultaneously with the rack 5,50. The support ring 54 also maintains a space 55 between the bottles 56 so as to reduce the risk of cross-contamination as illustrated in Figure 12.

Although the rack is described with reference to the embodiment illustrated in Figure 1 and 3, it will be appreciated that the rack may also be used with the any of the units illustrated in Figures 4 to 8.

Although embodiments of the invention have been shown and described, it will be appreciated by those persons skilled in the art that the foregoing description should be regarded as a description of preferred embodiments only and that other embodiments that fall within the scope of the appended claims are considered to form part of this disclosure.

## Claims

1. A washing unit for washing and/or sterilising a baby care component comprising a base (2) and a lid (3), the base having a bottom wall (9), a side wall (10) extending upwardly from the periphery of the bottom wall, wherein a line of closure between the base and the lid at least partially extends in a direction away from the bottom wall, and a rim (11), the rim (11) comprising a lower portion (12),
**characterised in that** the lower portion (12) of the rim (11) is positioned so that a baby care component is movable into and out of the unit in a direction substantially parallel to the bottom wall when the lid is removed from the base.

2. A unit according to claim 1, wherein the lid (3) comprises a top wall (15), and the distance between the bottom wall (9) and the closest section of the line of closure to the bottom wall is less than the distance between said closest section and the top wall of the lid.

3. A unit according to claim 1 or claim 2, wherein the line of closure is formed along a plane which extends at an oblique angle to the bottom wall (9) of the base (2).

4. A unit according to claim 1, further comprising engagement means extending along the line of closure such that the lid (3) is engagably mountable to the base (2), the lid being configured to be removed from the base in a direction parallel to the base once the lid is disengaged from the base.

5. A unit according to claim 4, wherein the side wall (10) of the base (2) has a rim (11) extending therearound and the lid (3) comprises a top wall (15) and a lid side wall (16) depending from the top wall, the lid side wall having a brim (17) which is configured to locate against the rim of the base when the lid is engagably mounted to the base.

6. A unit according to claim 5, wherein the lid side wall (16) extends circumferentially around the top wall (15) and the brim (17) is spaced from said top wall.

7. A unit according to claim 5, wherein the base (2) further comprises an upper wall (35) extending from an upper end of the base side wall (10) and spaced from the bottom wall (9), and an edge of the upper wall defines part of the line of closure.

8. A unit according to any of claims 4 to 7, wherein the engagement means is a labyrinth seal (43).

9. A unit according to any preceding claim, wherein the line of closure is a primary line of closure, and the side wall (10) of the base (2) further comprises an upper section (61) and a lower section (62), wherein a secondary line of closure between the upper section and the lower section extends around the side wall, such that the upper section is removable from the lower section.

10. A unit according to any of claims 1 to 8, wherein the side wall (10) of the base (2) is removably attachable to the bottom wall (9) of the base.

11. A unit according to any preceding claim, wherein the base (2) and the lid (3) together define a baby care component receiving space (4), and the unit further comprises a rack (5, 6) disposed in the baby care component receiving space which is rotatable in the space about a vertical axis extending perpendicular to the base.

## Patentansprüche

1. Wascheinheit zum Waschen und/oder Sterilisieren einer Säuglingspflegekomponente, mit einer Basis (2) und einem Deckel (3), wobei die Basis eine Bodenwand (9), eine sich von dem äußeren Rand der Bodenwand nach oben erstreckende Seitenwand (10), wobei sich eine Schließlinie zwischen der Basis und dem Deckel zumindest teilweise in einer Richtung von der Bodenwand weg erstreckt, sowie einen Rand (11) aufweist, wobei der Rand (11) einen unteren Teil (12) umfasst,
**dadurch gekennzeichnet, dass** der untere Teil (12) des Randes (11) so positioniert ist, dass eine Säuglingspflegekomponente in die und aus der Einheit in einer Richtung im Wesentlichen parallel zu der Bodenwand bewegbar ist, wenn der Deckel von der Basis entfernt wird.

2. Einheit nach Anspruch 1, wobei der Deckel (3) eine Deckwand (15) umfasst und der Abstand zwischen der Bodenwand (9) und dem nächstgelegenen Abschnitt der Schließlinie zu der Bodenwand geringer als der Abstand zwischen dem nächstgelegenen Abschnitt und der Deckwand des Deckels ist.

3. Einheit nach Anspruch 1 oder 2, wobei die Schließlinie entlang einer Ebene gebildet wird, die sich in einem schrägen Winkel zu der Bodenwand (9) der Basis (2) erstreckt.

4. Einheit nach Anspruch 1, die weiterhin Eingriffsmittel umfasst, die sich entlang der Schließlinie so erstrecken, dass der Deckel (3) an der Basis (2) einrastbar angebracht werden kann, wobei der Deckel so ausgeführt ist, dass er von der Basis in einer Richtung parallel zu der Basis entfernt wird, sobald der Deckel von der Basis getrennt wird.

5. Einheit nach Anspruch 4, wobei die Seitenwand (10) der Basis (2) einen sich um diese erstreckenden Rand (11) aufweist und der Deckel (3) eine Deckwand (15) und in Abhängigkeit der Deckwand eine Deckel-Seitenwand (16) umfasst, wobei die Deckel-Seitenwand eine Kante (17) aufweist, die so ausgeführt ist, dass sie gegen den Rand der Basis anliegt, wenn der Deckel an der Basis einrastbar angebracht ist.

6. Einheit nach Anspruch 5, wobei sich die Deckel-Seitenwand (16) umlaufend um die Deckwand (15) erstreckt und die Kante (17) von der Deckwand beabstandet ist.

7. Einheit nach Anspruch 5, wobei die Basis (2) weiterhin eine obere Wand (35) umfasst, die sich von einem oberen Ende der Seitenwand (10) der Basis aus erstreckt und von der Bodenwand (9) beabstandet ist, und wobei ein Rand der oberen Wand die Schließlinie definiert.

8. Einheit nach einem der Ansprüche 4 bis 7, wobei es sich bei den Eingriffsmitteln um eine Labyrinthdichtung (43) handelt.

9. Einheit nach einem der vorangegangenen Ansprüche, wobei die Schließlinie eine primäre Schließlinie ist, und wobei die Seitenwand (10) der Basis (2) weiterhin einen oberen Abschnitt (61) und einen unteren Abschnitt (62) umfasst, wobei sich eine sekundäre Schließlinie zwischen dem oberen Abschnitt und dem unteren Abschnitt so um die Seitenwand erstreckt, dass der obere Abschnitt von dem unteren Abschnitt entfernt werden kann.

10. Einheit nach einem der Ansprüche 1 bis 8, wobei die Seitenwand (10) der Basis (2) an der Bodenwand (9) der Basis entfernbar befestigt werden kann.

11. Einheit nach einem der vorangegangenen Ansprüche, wobei die Basis (2) und der Deckel (3) zusammen einen Aufnahmeraum (4) für Säuglingspflegekomponenten definieren, und wobei die Einheit weiterhin eine in dem Aufnahmeraum für Säuglingspflegekomponenten angeordnete Ablage (5, 6) umfasst, die in dem Raum um eine sich senkrecht zu der Basis erstreckende vertikale Achse drehbar ist.

## Revendications

1. Unité de lavage pour laver et/ou stériliser un article de puériculture comprenant une base (2) et un couvercle (3), la base possédant une paroi inférieure (9), une paroi latérale (10) s'étendant vers le haut depuis la périphérie de la paroi inférieure, dans laquelle une ligne de fermeture entre la base et le couvercle s'étend au moins partiellement dans une direction opposée à la paroi inférieure, et un rebord (11), le rebord (11) comprenant une partie inférieure (12),
**caractérisée en ce que** la partie inférieure (12) du rebord (11) est positionnée de telle sorte qu'un article de puériculture soit mobile à l'intérieur et en dehors de l'unité dans une direction sensiblement parallèle à la paroi inférieure, lorsque le couvercle est retiré de la base.

2. Unité selon la revendication 1, dans laquelle le couvercle (3) comprend une paroi supérieure (15), et la distance entre la paroi inférieure (9) et la section de la ligne de fermeture la plus proche de la paroi inférieure est inférieure à la distance entre ladite section la plus proche et la paroi supérieure du couvercle.

3. Unité selon la revendication 1 ou la revendication 2, dans laquelle la ligne de fermeture est formée le long d'un plan qui s'étend selon un angle oblique vers la paroi inférieure (9) de la base (2).

4. Unité selon la revendication 1, comprenant en outre des moyens de mise en prise s'étendant le long de la ligne de fermeture, de telle sorte que le couvercle (3) puisse être monté de manière à être en prise avec la base (2), le couvercle étant configuré pour être retiré de la base dans une direction parallèle à la base une fois que le couvercle est libéré de la base.

5. Unité selon la revendication 4, dans laquelle la paroi latérale (10) de la base (2) a un rebord (11) s'étendant autour d'elle, et le couvercle (3) comprend une paroi supérieure (15) et une paroi latérale de couvercle (16) dépendant de la paroi supérieure, la paroi latérale de couvercle ayant un bord (17) qui est configuré pour se placer contre le rebord de la base lorsque le couvercle est monté de manière à être en prise avec la base.

6. Unité selon la revendication 5, dans laquelle la paroi latérale du couvercle (16) s'étend circonférentiellement autour de la paroi supérieure (15) et le bord (17) est espacé de ladite paroi supérieure.

7. Unité selon la revendication 5, dans laquelle la base (2) comprend en outre une paroi supérieure (35) s'étendant à partir d'une extrémité supérieure de la paroi latérale de la base (10) et espacée par rapport à la paroi inférieure (9), et un bord de la paroi supérieure définit une partie de la ligne de fermeture.

8. Unité selon l'une quelconque des revendications 4 à 7, dans laquelle le moyen de mise en prise est un joint labyrinthe (43).

9. Unité selon l'une quelconque des revendications précédentes, dans laquelle la ligne de fermeture est une ligne principale de fermeture, et la paroi latérale (10) de la base (2) comprend en outre une section supérieure (61) et une section inférieure (62), dans laquelle une ligne de fermeture secondaire entre la section supérieure et la section inférieure s'étend autour de la paroi latérale, de telle sorte que la section supérieure puisse être retirée de la section inférieure.

10. Unité selon l'une quelconque des revendications 1 à 8, dans laquelle la paroi latérale (10) de la base (2) peut être attachée de manière amovible à la paroi inférieure (9) de la base.

11. Unité selon l'une quelconque des revendications précédentes, dans laquelle la base (2) et le couvercle (3) définissent ensemble un espace récepteur d'article de puériculture (4), et l'unité comprend en outre un support (5, 6) disposé dans l'espace récepteur d'article de puériculture qui peut tourner dans l'espace autour d'un axe vertical s'étendant perpendiculairement à la base.
